# EUROPEAN PATENT SPECIFICATION

(11) **EP 3 596 385 B1**
(45) Date of publication and mention of the grant of the patent: **09.06.2021**
(21) Application number: 18718507.9
(22) Date of filing: 16.03.2018
(51) Int. Cl.: F21V 5/00, C12M 1/00, F21V 8/00, F21K 9/61

(54) **DIFFUSER TUBE**
VERTEILERROHR
TUBE DIFFUSEUR

(30) Priority: 17.03.2017 IT 201700029945
(43) Date of publication of application: 22.01.2020
(73) Proprietor: BIOSYNTEX S.R.L., 91025 Paceco (TP) (IT)
(72) Inventor: GRILL, Joachim, I-91025 Paceco (Trapani) (IT); STAGL, Karl, I-91025 Paceco (Trapani) (IT); VINCENZI, Donato, I-44121 Ferrara (IT); LESCI, Isidoro Giorgio, I-48014 Castel Bolognese (Ravenna) (IT)
(74) Representative: Rigamonti, Dorotea
(86) International application number: PCT/IB2018/051762
(87) International publication number: WO 2018/167721

(56) References cited:
- EP-A1- 2 314 907
- WO-A2-2012/128547
- US-A- 5 617 497
- US-A1- 2012 057 350
- US-A1- 2014 029 264

## Description

The present invention relates to a light diffuser tube, characterized in that said light tube is coated with an OLF and allows light to exit at an almost constant intensity over the entire length of said tube.

### Background art

Phototrophic and mixotrophic algae cultures require a certain amount of light radiation to promote the photosynthesis process and metabolize CO₂ while releasing O₂. Under particular nourishment and light regimes, for these cultures, a proliferation of the algal population is observed, such as to be employed for the extraction of chemicals, active ingredients to be used, for example, in fuel, cosmetics, pharmaceutical, food and of fertilizers industries.

The light radiation with which algal growth is promoted is commonly provided by the sun. Such light may be direct, i.e., directly hit the culture medium, or indirect. In this latter case, the light radiation is concentrated through optical devices (mirrors or lenses) and possibly conveyed through optical fibers or light tubes towards the culture medium.

The use of indirect light has several advantages, including the possibility of providing a level of optimal light radiation intensity for algal growth, without having to filter or absorb part of the excess light radiation.

WO2015192159A1 describes a solar light concentration system used in photobioreactors. The optical concentration element consists of a parquet of Fresnel lenses, while optical fibers conjunction elements are placed on the focal plane of such lenses.

Optical fibers employ the principle of total internal reflection to trap light rays and guide them along the path of the fiber itself. Only the portion of light which points on the inlet surface of the optical fiber at an angle lower than a critical angle called "acceptance angle" is refracted by the optical material of the fiber itself and undergoes the total internal reflection phenomenon. The light radiation entering inside the fiber at an angle greater than the acceptance angle is rapidly diffused or absorbed by the cladding of the fiber itself and is not guided along the optical path.

Only the light radiation within the acceptance angle is effectively guided by the optical fiber towards the culture medium and, due to the refraction phenomenon occurring at the interface between the fiber outlet surface and the external medium, diverges into a beam known as a "light cone", the width thereof is greater with respect to the angular acceptance.

The angular divergence of the light radiation exiting the optical fibers is such as to make a further optical element, which redistributes the light so as to uniformly illuminate the culture medium, typically necessary.

The optic fiber bundle outlet surface is typically located at the upper part of a "light tube" which illuminates the culture medium. The radiation from the optical fiber outlet surface almost completely exits the light tube in the first meter of length.

In the background art, Optical Light Films (OLFs) are widely used in light diffusers, characterized in that they have one of the two surfaces covered by microprisms which, by virtue of the total internal reflection phenomenon, trap the light radiation therein and guide it along the film. Only the light radiation pointing on an OLF at an angle of incidence greater than 64° with respect to the line perpendicular to the film remains trapped inside the film itself, while the light radiation pointing at lower angles undergoes refraction but manages to exit the film at essentially random angles. Due to the direction of the microprism corners, the OLF is a highly anisotropic optical element. The direction of the microprisms corners identifies the preferential "transport" direction of the light radiation and is usually parallel to the axial direction of the light tube.

The light radiation trapped inside the OLF undergoes a minimum attenuation due to the absorption of the material but is not reduced in intensity due to the multiple reflections due to the total internal reflection phenomenon.

One of the essential issues arising from the use of OLF is the extraction of light radiation. This typically occurs by inserting an "extractor" inside the light tube, i.e., a reflecting or diffusing optical element which "breaks" the symmetry of the system and which allows the light redirected towards the OLF to have an angle of incidence lower than the critical angle of 64°. Such extractors are typically long cones which are inserted at the bottom of the light tube and which have a height substantially parallel to the optical axis of the cylinder. In practice, and in particular in the use in conjunction with light tubes for photobioreactors, the extractors would have a very high form factor, i.e., a very high height to base side size ratio. The construction of high-form factor extractors is a complex and burdensome operation and proves to be economically inconvenient.

The need for a solution which allows to significantly increase the uniformity of the light tube emittance profile without affecting the initial investment and having a limited impact on the cost of the system is strongly felt

The document US 2014/029264 A1 shows the preamble of claim 1.

### Description of the invention

The present invention relates to a light diffuser tube, characterized in that said light tube is coated with OLF and allows light to exit at an almost constant intensity over the entire length of said tube.

### Description of the drawings

Figure 1: perspective view of a light tube according to the present invention.
Figure 2: view of a further embodiment of the light tube according to the present invention.
Figure 3: view of a further embodiment of the light tube according to the present invention.
Figure 4: view of a further embodiment of the light tube according to the present invention.
Figure 5: false-color graph of the emittance profile of the surface of the light tube, following the use of the diffuser according to the present invention (panel A) or of light tubes known in the background art (panel B).

### Detailed description of the invention:

The present invention relates to a light diffuser which uses reflecting films employing the total internal reflection phenomenon (OLFs), characterized in that said OLFs are shaped so as to allow to effectively trap and extract light radiation without the use of a high-form factor extractor element.

The OLF is an essentially anisotropic optical element and is characterized by a preferential light radiation transmission direction. In order to preserve the light transmission features of the OLF, the absence of interruptions of the film in the longitudinal direction (parallel to the corners of the microprisms) is essential. Each interruption of the film in this direction results in the extraction of a part of the light radiation trapped.

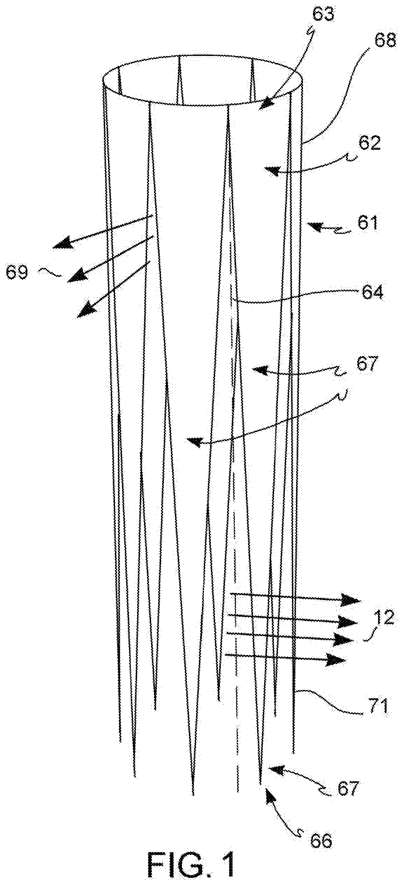

In the solution proposed herein, the OLF is arranged so as to cover the internal surface of the light tube and it coats a portion of the circumference of the tube gradually decreasing when moving away from the light source, constituted by the optical fiber outlet plane. With reference to Figure 1, the light tube (61) is covered with the OLF (62). Said light tube (61) has an upper region (68) and a lower region (71). Said covering is given by OLF (62) essentially shaped as an isosceles triangle, where the base (65) is applied to the inlet of the light fibers in said light tube (61), in the upper region (68) and the vertex (66) is essentially at the outlet of said fibers from said light tube, in the lower region (71). The geometry of said OLFs leaves, therefore, uncovered portions (64) on said light tube (61), in which also said uncovered portions (64) are essentially shaped as an isosceles triangle, in which the greatest height (67) of said triangles, which are the uncovered portions (64), is essentially parallel to the direction of the microprisms of the OLF and parallel to the optical axis of the light tube (61).

The portion (69) of the light flux, emitted from the upper region (68) of said light tube, is characterized by a greater irradiance, but exits from a reduced area due to the reduced width of the openings of an essentially triangular shape (64). In the lower region (71) the portion (72) of the light flux emitted by the diffuser is characterized by a relatively low irradiance, but is distributed over a significantly larger area, giving rise to an average emittance substantially equal to that present in the upper region (68).

In a preferred embodiment, said uncovered portions (64) are about 4 for each light tube, or 6, or 8, preferably 8 or more.

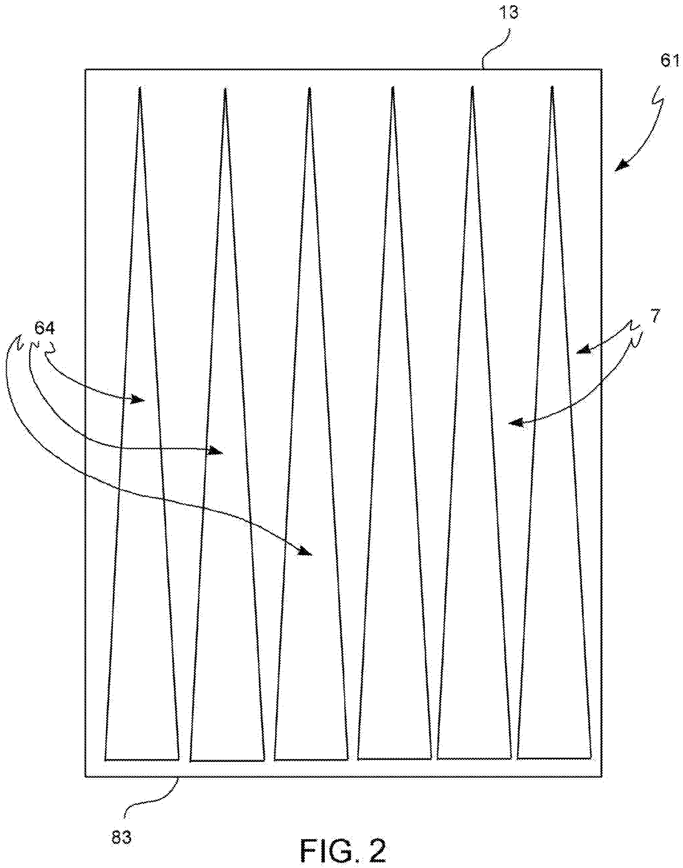

In a further embodiment, diagrammatically shown in Figure 2, the light tube (61) has the openings of an essentially triangular shape (64) and, along the circumference of said light tube, a frame (83) of OLF material. Said frame (83) gives strength and structure to the diffuser itself.

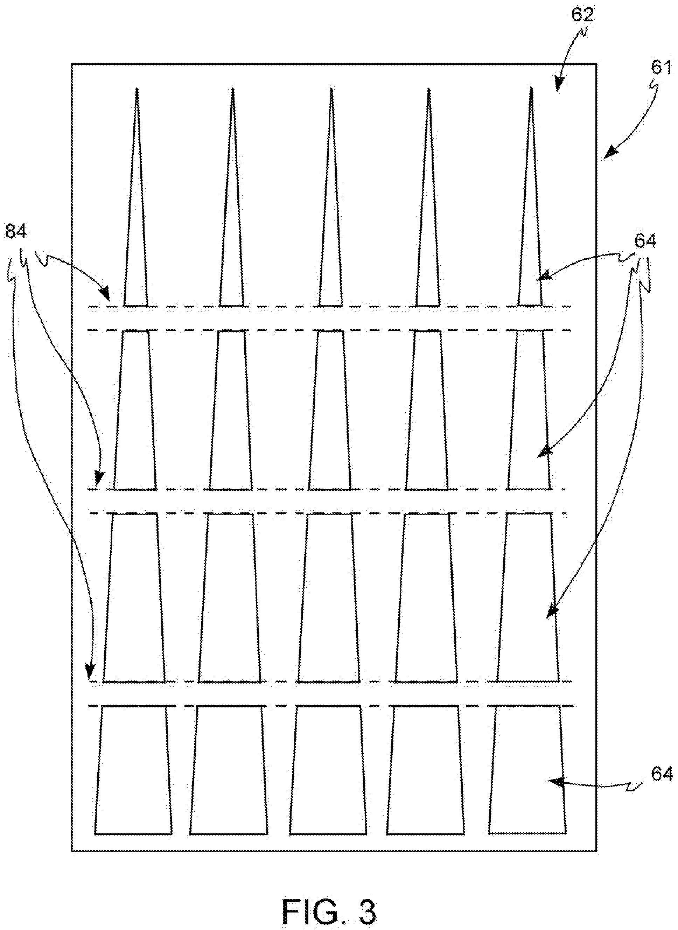

In a further embodiment, with reference to Figure 3, on said OLFs (62) there are a series of openings (64) of a triangular or trapezoidal shape, crossed by continuous strips (84) of OLF material. Said continuous strips (84) are therefore arranged along the entire length of said light tube, parallel to the base thereof. Such continuous strips (84) give greater mechanical stability to the diffuser and contribute to facilitating the installation thereof inside the light tubes. The openings (64) on the surface of the diffuser are, therefore, in this particular embodiment, of a triangular or trapezoidal shape.

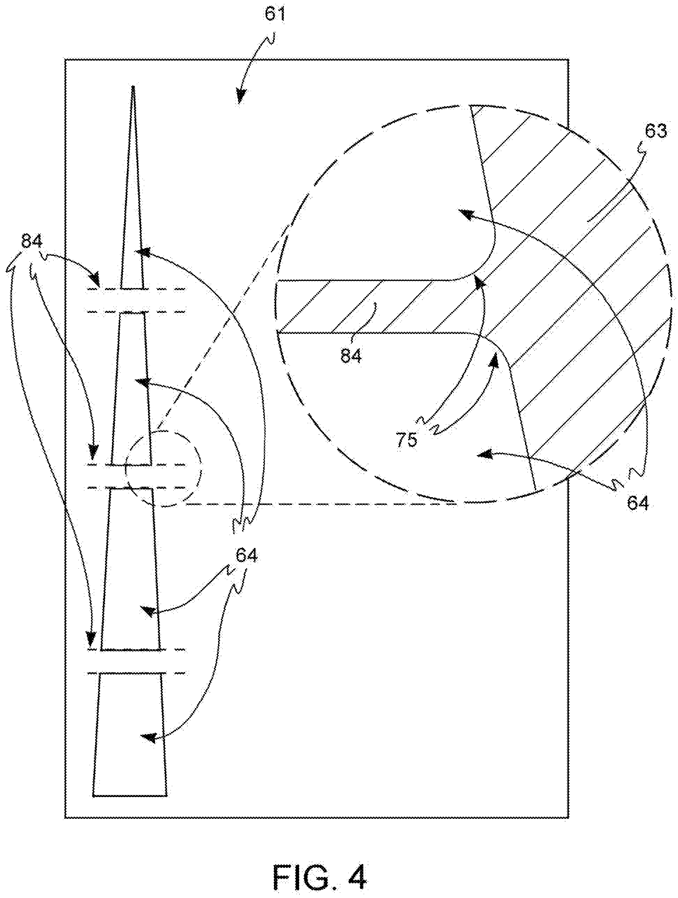

Preferably, as diagrammatically shown in Figure 4, said openings (64), interrupted by continuous strips (84) of OLF material which identify open regions of a triangular or trapezoidal shape, have rounded corners (85). Said rounded corners give greater mechanical stability to the OLF.

Due to the reduced percentage of light tube surface covered with the OLF and the particular distribution of said film on the light tube, a gradually greater portion of light may exit the light tube and illuminate the culture medium.

The distribution of the film on the light tube ensures that, in the region of greatest light intensity, i.e., at the inlet of the optical fibers in the light tube, only a small portion of the light beam is extracted.

When moving away from the inlet plane of the optical fibers in the axial direction, the circumference portion of the light tube affected by said openings increases essentially linearly with the distance, allowing the radiation trapped inside the light tube to meet a greater surface through which to exit. The solution proposed herein surprisingly allows to maintain the optical power extracted from the light tube almost constant when moving away from the plane of the optical fibers towards the bottom of the light tube.

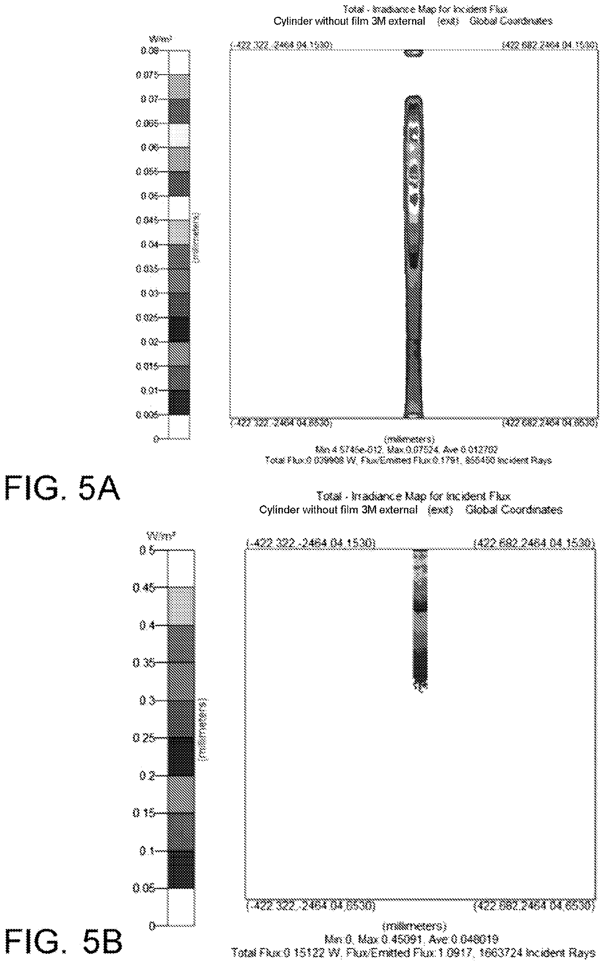

As shown by the graph in Figure 5, which shows the emittance profile of the light tube surface following the use of the diffuser of the present invention (panel A), the light intensity exiting from the light tube (61) proves to be uniform as compared to the solution in which the OLF, shaped according to the principles described in this patent application (panel B), is not present.

As mentioned above, the light tubes are often characterized by a very high form factor, where high form factor means a high length to diameter ratio of said tube, and this provides for the installation of an OLF complex therein. The presence of long empty areas (64) in the OLF is a practical issue which may be overcome by using an OLF frame (83) holding the region characterized by the openings (64) together. Such frame gives structure and mechanical stability to the film and makes the installation of the film inside the light diffuser easier.

For light tubes having a form factor higher than 10, it proves to be particularly advantageous that the openings of an essentially triangular or trapezoidal shape (64) are crossed by continuous strips (84) of OLF material. This solution also contributes to giving mechanical rigidity and structure to the optical diffuser and makes the installation thereof inside the light tube easier. The area covered by said continuous strips (84) of OLF material is substantially smaller than the overall area of the openings (64) and does not significantly alter the light-extraction properties of the diffuser.

Said openings (64) may have sharp corners, which weaken the structure of the OLF and may sometimes generate fracture lines at said corners. In order to increase the mechanical stability of the diffuser and increase the simplicity of installation inside the light diffuser tube, such sharp corners, as in the embodiment of Figure 4, are rounded (85). The introduction of a radius of curvature - even if small - allows to distribute stress and to drastically reduce the probability of generating a fracture. From the optical point of view, the introduction of the continuous strips (84) or of the radii of curvature (85) does not significantly affect the optical properties of the diffuser.

Only a small portion of the light flux emitted by the bundle of optical fibers, almost parallel to the optical axis of the system, is not extracted from the light tubes (1) because it does not reach the surface of the OLF (62), nor the uncovered portions (64). In order to extract also said light radiation, in an embodiment, at the end of the light tube (1) a light extraction cone is placed which redirects the light radiation towards the outside of said light tube (1) and therefore towards the culture medium. The use of cones for extracting light radiation from light guides is a technique known to those skilled in the art, but usually the height of the extraction cone must be substantially comparable with the length of the light tube. This requires that the material of which said cone is constituted has a high structural rigidity and this increases the intrinsic cost of said cone and increases the construction difficulty.

On the other hand, if the light diffuser of the present patent application is adopted, the height of the light cone is highly reduced, equal to fractions of the light cone diameter, and this makes the employment of reflecting materials with low intrinsic cost possible, such as cold drawn, folded-up metal sheets or other solutions which do not require high structural rigidity.

This allows to ensure a high optical extraction efficiency and a limited intrinsic and working cost, as compared to that needed in the absence of the light diffuser of the present invention.

## Claims

1. A light tube (61) covered with Optical Light Film (62) (OLF), said tube having an upper inlet region (68) for optical fibers and a lower outlet region (71) for said optical fibers, wherein said film is a film having one of the two surfaces covered with microprisms, **characterized in that** said OLFs (62) are essentially shaped as an isosceles triangle, wherein the base (65) of said triangle is at the inlet of the optical fibers in said light tube (61) and the vertex (66) is essentially at the outlet of said fibers from said light tube, and that said OLFs leave uncovered portions (64) on said light tube (61), wherein said uncovered portions (64) are also essentially shaped as an isosceles triangle, wherein the greatest height (67) of said triangles, which are the uncovered portions (64), is essentially parallel to the direction of the microprisms of the OLF and parallel to the optical axis of the light tube (61).

2. A light tube according to claim 1, further comprising, along the circumference of said light tube, a frame (83) of OLF material holding said openings (64) together.

3. A light tube according to claim 1 or 2, further comprising continuous strips (84) of OLF material placed along said light tube, parallel to the base thereof.

4. A light tube according to one of claims 1 to 3, wherein said openings (64) have rounded corners (85).

5. A light tube according to one of claims 1 to 4, wherein said uncovered portions (64) are about 4 for each light tube, or 6, or 8, preferably they are 8 or more.

## Patentansprüche

1. Lichtröhre (61), die mit einem optischen Lichtfilm (62) (OLF) bedeckt ist, wobei die Röhre einen oberen Einlassbereich (68) für optische Fasern und einen unteren Auslassbereich (71) für die optischen Fasern aufweist, wobei bei dem Film eine der beiden Oberflächen mit Mikroprismen bedeckt ist, **dadurch gekennzeichnet, dass** die OLFs (62) im Wesentlichen als gleichschenkliges Dreieck geformt sind, wobei die Basis (65) des Dreiecks am Einlass der optischen Fasern in die Lichtröhre (61) liegt und die Spitze (66) im Wesentlichen am Auslass von den Fasern aus der Lichtröhre liegt und die OLFs unbedeckte Abschnitte (64) auf der Lichtröhre (61) zurücklassen, wobei die unbedeckten Abschnitte ebenfalls im Wesentlichen als ein gleichschenkliges Dreieck geformt sind, wobei die größte Höhe (67) der Dreiecke, welche die unbedeckten Abschnitte (64) sind, im Wesentlichen parallel zur Richtung der Mikroprismen des OLFs und parallel zur optischen Achse der Lichtröhre (61) ist.

2. Lichtröhre nach Anspruch 1, ferner umfassend einen Rahmen (83) aus OLF-Material entlang des Umfangs der Lichtröhre, der die Öffnungen (64) zusammenhält.

3. Lichtröhre nach Anspruch 1 oder 2, die ferner kontinuierliche Streifen (84) aus OLF-Material umfasst, die entlang der Lichtröhre parallel zu deren Basis angeordnet sind.

4. Lichtröhre nach einem der Ansprüche 1 bis 3, wobei die Öffnungen (64) abgerundete Ecken (85) aufweisen.

5. Lichtröhre nach einem der Ansprüche 1 bis 4, wobei die unbedeckten Abschnitte (64) etwa 4 oder 6 oder 8, vorzugsweise 8 oder mehr, für jede Lichtröhre sind.

## Revendications

1. Conduit de lumière (61) recouvert avec un film d'éclairage optique (Optical Light Film : OLF) (62), ledit tube comportant une région d'entrée supérieure (68) pour des fibres optiques et une région de sortie inférieure (71) pour lesdites fibres optiques, dans lequel ledit film est un film ayant une des deux surfaces recouverte avec des microprismes, **caractérisé en ce que** lesdits OLF (62) sont formés essentiellement sous la forme d'un triangle isocèle, dans lequel la base (65) dudit triangle est à l'entrée des fibres optiques dans ledit conduit de lumière (61) et le sommet (66) est essentiellement à la sortie desdites fibres depuis ledit conduit de lumière, et
lesdits OLF laissent des parties non recouvertes (64) sur ledit conduit de lumière (61), dans lequel lesdites parties non recouvertes (64) sont également formées essentiellement sous la forme d'un triangle isocèle, dans lequel la plus grande hauteur (67) desdits triangles, qui sont les parties non recouvertes (64), est essentiellement parallèle à la direction des microprismes de l'OLF et parallèle à l'axe optique du conduit de lumière (61).

2. Conduit de lumière selon la revendication 1, comprenant en outre, le long de la circonférence dudit conduit de lumière, un cadre (83) de matériau OLF maintenant lesdites ouvertures (64) ensemble.

3. Conduit de lumière selon la revendication 1 ou 2, comprenant en outre des bandes continues (84) de matériau OLF placées le long dudit conduit de lumière, parallèles à la base de celui-ci.

4. Conduit de lumière selon l'une des revendications 1 à 3, dans lequel lesdites ouvertures (64) ont des coins arrondis (85).

5. Conduit de lumière selon l'une des revendications 1 à 4, dans lequel lesdites parties non recouvertes (64) sont environ 4 pour chaque conduit de lumière, ou 6, ou 8, de préférence elles sont 8 ou plus.
